# EUROPEAN PATENT SPECIFICATION

(11) **EP 3 988 119 B1**
(45) Date of publication and mention of the grant of the patent: **08.10.2025**
(21) Application number: 20827931.5
(22) Date of filing: 22.05.2020
(51) Int. Cl.: A61K 47/52, A61K 47/69, A61P 31/04, A61K 47/02, A61K 9/08, A61K 9/19, A61K 31/43, A61K 31/44, A61K 45/06

(54) **ORALLY-ADMINISTERED PHARMACEUTICAL COMPOSITION FOR ERADICATING ANTIBIOTIC-RESISTANT HELICOBACTER PYLORI, COMPRISING COMPLEX OF NON-ABSORBABLE ANTIBIOTIC AND CLAY MINERAL**
ORAL VERABREICHBARE PHARMAZEUTISCHE ZUSAMMENSETZUNG ZUR ERADIKATION VON ANTIBIOTIKARESISTENTEM HELICOBACTER PYLORI MIT EINEM KOMPLEX AUS NICHT-ABSORBIERBAREM ANTIBIOTIKUM UND TONMINERAL
COMPOSITION PHARMACEUTIQUE À ADMINISTRATION ORALE POUR ÉRADIQUER HELICOBACTER PYLORI RÉSISTANT AUX ANTIBIOTIQUES, COMPRENANT UN COMPLEXE D'ANTIBIOTIQUE NON ABSORBABLE ET DE MINÉRAL ARGILEUX

(30) Priority: 20.06.2019 KR 20190073709
(43) Date of publication of application: 27.04.2022
(73) Proprietor: KOREA INSTITUTE OF GEOSCIENCE AND MINERAL RESOURCES, Yuseong-gu Daejeon 34132 (KR)
(72) Inventor: KIM, Jae Hwan, Pohang-si Gyeongsangbuk-do 37571 (KR); KANG, Il-Mo, Seoul 07528 (KR); SONG, Young Goo, Seoul 06714 (KR); ROH, Ki Min, Daejeon 34061 (KR); SONG, Yungoo, Seoul 02701 (KR); SEO, Sung Man, Pohang-si Gyeongsangbuk-do 37883 (KR); KIM, Dae Young, Geumsan-gun Chungcheongnam-do 32722 (KR)
(74) Representative: Bardehle Pagenberg Partnerschaft mbB Patentanwälte Rechtsanwälte
(86) International application number: PCT/KR2020/006721
(87) International publication number: WO 2020/256295

(56) References cited:
- FR-A1- 2 677 253
- KR-B1- 101 541 876
- KR-B1- 101 541 876
- KR-B1- 102 054 341
- SU JIN JEONG ET AL: "Gentamicin-intercalated smectite as a new therapeutic option for Helicobacter pylori eradication", JOURNAL OF ANTIMICROBIAL CHEMOTHERAPY, vol. 73, 12 February 2018 (2018-02-12), GB, pages 1324 - 1329, XP055696917, ISSN: 0305-7453, DOI: 10.1093/jac/dky011
- LEE KYOUNG HWA ET AL: "Can Aminoglycosides Be Used as a New Treatment for Helicobacter pylori ? In vitro Activity of Recently Isolated Helicobacter pylori", vol. 51, no. 1, 1 January 2019 (2019-01-01), pages 10, XP055773403, ISSN: 2093-2340, Retrieved from the Internet <URL:https://synapse.koreamed.org/DOIx.php?id=10.3947/ic.2019.51.1.10> DOI: 10.3947/ic.2019.51.1.10
- KYOUNG HWA LEE ET AL: "2449. Validation of in vitro activity of aminoglycosides against recently isolated Helicobacter pylori for commercialization of gentamicin-intercalated smectite hybrid as a new therapeutic agent", OPEN FORUM INFECTIOUS DISEASES, vol. 5, no. suppl.1, 1 January 2018 (2018-01-01), pages S733, XP055696927, DOI: 10.1093/ofid/ofy210.2102
- JEONG, S. J. ET AL.: "Gentamicin-intercalated smectite as a new therapeutic option for Helicobacter pylori eradication", J. ANTIMICROB. CHEMOTHER, vol. 73, 12 February 2018 (2018-02-12), pages 1324 - 1329, XP055696917, DOI: 10.1093/jac/dky011
- SHAO, Y. ET AL.: "Antibiotic resistance of Helicobacter pylori to 16 antibiotics in clinical patients", J. CLIN. LAB. ANAL. ELECTRONIC, vol. 32, no. 4, 6 October 2017 (2017-10-06), pages 1 - 5, XP055696915, DOI: 10.1002/jcla.22339
- LEE, KYOUNG HWA, PARK SOON YOUNG, JEONG SU JIN, JUNG DA HYUN, KIM JIE-HYUN, JEONG SEOK HOON, KANG IL-MO, SONG YOUNG GOO: "Can aminoglycosides be used as a new treatment for Helicobacter pylori? In vitro activity of recently isolated Helicobacter pylori", INFECT. CHEMOTHER, vol. 51, no. 1, March 2019 (2019-03-01), pages 10 - 20, XP055773403, DOI: 10.3947/ic.2019.51.1.10
- LEE, K. H. ET AL.: "Validation of In Vitro Activity of Aminoglycosides Against Recently Isolated Helicobacter pylori for Commercialization of Gentamicin-Intercalated Smectite Hybrid as a New Therapeutic Agent", IDWEEK 2018, 3 October 2018 (2018-10-03), XP055696927, DOI: 10.1093/ofid/ofy210.2102
- LEE, K. H. ET AL.: "fecal microbiome changes after helicobacter pylori eradication with smectite-gentamicin hybrid in mouse model", CONFERENCE, 4 April 2019 (2019-04-04)

## Description

### [Technical Field]

The present disclosure relates to an orally-administered pharmaceutical composition for eradicating Helicobacter pylori comprising a complex of a non-absorbable antibiotic and a clay mineral. Further, the present disclosure relates to an orally-administered kit for eradicating Helicobacter pylori comprising a complex of a non-absorbable antibiotic and a clay mineral.

### [Background Art]

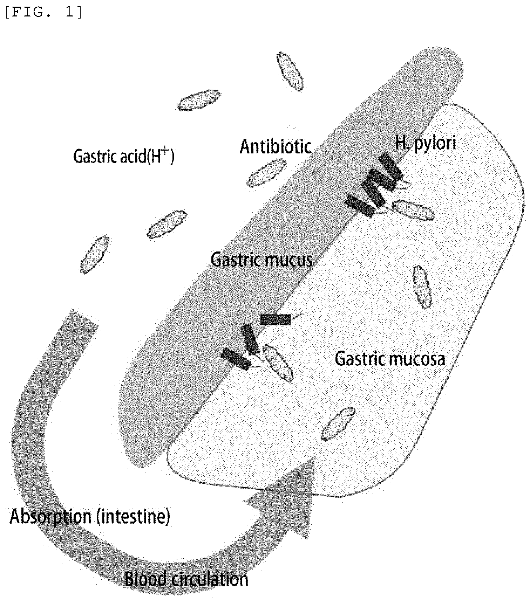

H. pylori is gram-negative microaerophilic spiral bacillus that affects the gastric mucosa and may be found to be attached to the epithelial cells on the human stomach. Drug-resistant H. pylori strains are the most common cause of treatment failure. Approximately 50% of the world's population is positive for H. pylori, and Developing countries have the prevalence of 80% to 90% and advanced countries have the prevalence of 35% to 40%. After the

recommendations on the treatment for eradicating H. pylori were first announced in Korea in 1988, a standard triple therapy that has been recommended for primary treatment so far is a method of combining and administering clarithromycin, amoxicillin, and a gastric acid-suppressive agent. The eradication effect of the standard triple therapy is currently very low about 70%, but a superior therapy to the existing triple therapy is not yet developed, and thus, despite the low eradication effect, the triple therapy has been recommended as the primary treatment (Korean J Gastroenterol 2013;62:3-26). As such, conventional therapies including the standard triple therapy are subjected to a process in which an orally-administered antibiotic is dissolved in the stomach, absorbed in the intestine, moves through blood vessels, and finally released to the gastric mucosa to eradicate H. pylori(FIG. 1). However, since the administered antibiotic is absorbed in the intestine and then released to the gastric mucosa to eradicate H. pylori, the antibiotic is systemically exposed to cause the risk of side effects and the burden of patients by the antibiotic.

Smectite is a leaflike silicate mineral constituting one unit layer (2:1 layer) by combining a tetrahedral sheet consisting of Si, Al, and Fe with two octahedral sheets consisting of Al, Mg, and Fe up and down in a sandwich shape. The smectite unit layer has a negative charge, which is generated when tetrahedral Si having a tetravalent positive charge is isomorphic-substituted to Al or Fe having a trivalent positive charge or octahedral Al or Fe³⁺ having a trivalent positive charge is isomorphic-substituted to Mg or Fe²⁺ having a bivalent positive charge. Cations are induced between unit layers through a negative charge generated from the unit layer, which means that smectite may be used as a drug carrier. Therefore, the use of smectite as a drug delivery vehicle has recently attracted great attention, and many studies have been reported on smectite hybrids into which drugs are inserted for controlled delivery and release of donepezil, lincomycin, chlorhexidine acetate, and tetracycline.

Aminoglycosides antibiotics are typical antibiotics used for treating gram-negative bacteria, but can be used only as injections without oral drugs, and thus, has not been used for treatment of eradicating **H.** pylori. In some studies, in evaluation of in-vitro activity of aminoglycosides on H. pylori, it was confirmed that a low minimum inhibition concentration was maintained. Among tested aminoglycosides, gentamicin, tobramycin, and netilmicin are the most active and have MIC90 and MIC50 values of 0.25 to 0.5 and 0.125 to 1.00 mg/L, respectively (Brenciaglia MI, Fornara AM, Scaltrito MMet al. Activity of amoxicillin, metronidazole, bismuth salicylate and six aminoglycosides against Helicobacter pylori. JChemother 1996; 8: 52-4). However, since the aminoglycosides are almost not absorbed in the gastrointestinal tract when orally administered, the aminoglycosides have been currently used only parenterally (Turnidge J. Pharmacodynamics and dosing of aminoglycosides. Infect Dis Clin NorthAm2003; 17: 503-28). Further the efficacy of gentamicin-intercalated smectite hybrid (S-GEN) -based treatment regimens in a murine model of H. pylori infection is studied(SU JIN JEONG ET AL: "Gentamicin-intercalated smectite as a new therapeutic option for Helicobacter pylori eradication",JOURNAL OF ANTIMICROBIAL CHEMOTHERAPY, vol. 73, 12 February 2018 (2018-02-12), pages 1324-1329). The question is asked "Can Aminoglycosides Be Used as a New Treatment for Helicobacter pylori? In vitro Activity of Recently Isolated isolated Helicobacter pylori" (LEE KYOUNG HWA ET AL,INFECTION & CHEMOTHERAPY,vol. 51, no. 1 1 January 2019 (2019-01-01), page 10).

Further, in actual in-vivo evaluation, it has been not reported that the aminoglycosides antibiotics exhibited an effect of eradicating H. pylori.

Therefore, the present inventors have studied a H. pylori eradicating agent which was orally administered and then effectively eradicated H. pylori in the gastrointestinal tract, confirmed that a composition for eradicating H. pylori of the present disclosure was not absorbed in the intestine and released from the stomach through blood vessels, but orally administered and then the antibiotic reaching to the stomach through the esophagus was attached to the gastric mucus layer to effectively eradicate H. pylori, and then completed the

### [Disclosure]

### [Technical Problem]

An object of the present disclosure is to provide a composition for eradicating H. pylori which is orally administered and then is applied and acts on the gastric mucosa.

### [Technical Solution]

In order to achieve the object, the present disclosure provides an orally-administered pharmaceutical composition for eradicating clarithromycin-resistant Helicobacter pylori comprising a complex of a non-absorbable antibiotic and a clay mineral, a β-lactam antibiotic, and a gastric acid-suppressive agent. Further, the present disclosure provides an orally-administered kit for eradicating clarithromycin-resistant

Helicobacter pylori comprising a complex of a non-absorbable antibiotic and a clay mineral.

### [Advantageous Effects]

According to the present disclosure, since the pharmaceutical composition and the kit include a plate-like clay carrier, it is possible to be applied on the gastric mucosa and enable targeted therapy on the gastric mucosa. In addition, there are advantages of reducing the antibiotic destruction in an intra-gastric environment with low acidity and minimizing the impact on intestinal bacteria.

### [Description of Drawings]

FIG. 1 illustrates a process of eradicating H. pylori according to a conventional triple therapy.
FIG. 2 illustrates a process of eradicating H. pylori by a complex of a non-absorbable antibiotic and a clay mineral of the present disclosure.
FIG. 3 illustrates the activity of eradicating Helicobacter pylori of a gentamicin-smectite complex (S-GEN). Upper left disk: Smectite, Upper right disk: S-GEN, Lower disk: gentamicin.
FIG. 4 illustrates the activity of eradicating Helicobacter pylori of a netilmicin-smectite complex (S-NET). Left disk: Smectite, Right disk: S-NET
FIG. 5 illustrates a protocol for identifying an anti-H. pylori pharmaceutical effect in vivo. The protocol includes inoculation of H. pylori, infection development, and therapy in C57BL/6 mice.
FIG. 6 illustrates gastric mucosal tissues and PCR results after studying a therapeutic effect of H. pylori infection using Groups 1 to 8.
FIG. 7 illustrates resistant rates of H. pylori on antibiotics.
FIG. 8A illustrates a result of comparing in-vitro antibacterial effects of a CLR disk and an S-GM disk in a clarithromycin-resistant strain.
FIG. 8B illustrates a result of comparing in-vitro antibacterial effects of a CLR disk and an S-GM disk in a clarithromycin-resistant strain.

### [Best Mode for the Invention]

The present disclosure provides an orally-administered pharmaceutical composition for eradicating clarithromycin-resistant Helicobacter pylori comprising a complex of a non-absorbable antibiotic and a clay mineral, a β-lactam antibiotic, and a gastric acid-suppressive agent.

The non-absorbable antibiotic is gentamicin.

The clay mineral is a smectite-group clay mineral namely montmorillonite, bentonite, beidellite, nontronite, saponite, or hectorite.

In the pharmaceutical composition, the complex of the non-absorbable antibiotic and the clay mineral are orally administered to reach the stomach through the esophagus and then attached to a gastric mucosal layer to release the antibiotic.

The pharmaceutical composition may have the urease inhibitory activity.

The pharmaceutical composition is a pharmaceutical composition for preventing or treating gastrointestinal diseases caused by clarithromycin-resistant Helicobacter pylori.

Further, the present disclosure provides an orally-administered kit for eradicating antibiotic-resistant Helicobacter pylori comprising a complex of a non-absorbable antibiotic and a clay mineral.

The kit comprises a β-lactam antibiotic.

The kit comprises a gastric acid-suppressive agent.

The antibiotic-resistant Helicobacter pylori is clarithromycin-resistant Helicobacter pylori.

### [Mode for the Invention]

The present disclosure relates to an orally-administered pharmaceutical composition for eradicating clarithromycin-resistant Helicobacter pylori comprising a complex of a non-absorbable antibiotic and a clay mineral as claimed.

Further, the present disclosure relates to an orally-administered pharmaceutical composition for eradicating antibiotic-resistant Helicobacter pylori comprising a complex of a non-absorbable antibiotic and a clay mineral.

Further, the present disclosure relates to an orally-administered kit for eradicating Helicobacter pylori comprising a complex of a non-absorbable antibiotic and a clay mineral.

Further, the present disclosure relates to an orally-administered kit for eradicating clarithromycin-resistant Helicobacter pylori comprising a complex of a non-absorbable antibiotic and a clay mineral.

Further, the present disclosure relates to a method for eradicating clarithromycin-resistant Helicobacter pylori comprising administering the pharmaceutical composition of the present disclosure to a subject.

Further, the present disclosure relates to a method for eradicating clarithromycin-resistant Helicobacter pylori comprising administering the pharmaceutical composition of the present disclosure to a subject.

Further, the present disclosure relates to a method for preventing or treating diseases caused by clarithromycin-resistant Helicobacter pylori comprising administering the pharmaceutical composition of the present disclosure to a subject.

Hereinafter, the present disclosure will be described in detail.

### Non-absorbable antibiotic

The non-absorbable antibiotic of the present disclosure is orally administered in the form of the complex with the clay mineral and then applied on the gastric mucosa when reaching the stomach through the esophagus. The antibiotic is released from the complex applied on the gastric mucosa to eradicate clarithromycin-resistant H. pylori.

The non-absorbable antibiotic of the present disclosure is not absorbed but released to reduce antibiotic side effects and the burden of patients when the complex is orally administered and not used for eradicating H. pylori in the stomach. Further, in this case, the non-absorbable antibiotic of the present disclosure is the complex form binding to the clay mineral to have a low effect on intestinal bacteria and minimize the antibiotic destruction under gastric acid conditions.

The non-absorbable antibiotic of the present disclosure is an aminoglycoside-based compound.

The aminoglycoside-based compound of the present disclosure is gentamicin.

### Clay mineral

In general, a clay mineral has a layered structure, that is, a plate-like structure in which crystal units formed by combining silica sheets and alumina sheets are stacked, and in a clay mineral having interlayer expansibility among these clay minerals, since the binding force between the crystal units is weak without hydrogen bonds between the crystal units, moisture is introduced between the crystal units to be expanded. Therefore, it is possible to easily introduce even ions having relatively large sizes between the crystal units of the clay mineral having interlayer expansibility. Meanwhile, in the clay mineral having interlayer expansibility, tetrahedral Si having a tetravalent positive charge is isomorphic-substituted to Al or Fe having a trivalent positive charge or octahedral Al or Fe³⁺ having a trivalent positive charge is isomorphic-substituted to Mg or Fe²⁺ having a bivalent positive charge to generate a negative layer charge, but cations such as calcium ions (Ca²⁺), magnesium ions (Mg²⁺), sodium ion (Na⁺), potassium ions (K⁺), and the like are bound between the layers or on the surface to have entirely electrical neutrality.

The clay mineral of the present disclosure is a clay mineral which has a plate-like structure, specifically, interlayer expansibility, and may be used as a carrier by inserting an antibiotic into the clay mineral. The clay mineral of the present disclosure is smectite-based minerals, namely montmorillonite or bentonite, beidellite, nontronite, saponite, hectorite, and the like.

### Complex of non-absorbable antibiotic and clay mineral

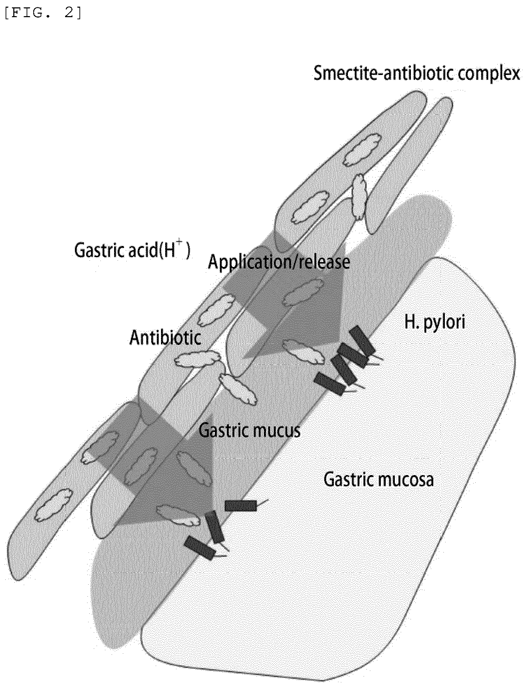

In the complex of the non-absorbable antibiotic and the clay mineral as claimed, the clay mineral is used as a delivery system, that is, a carrier for delivering the non-absorbable antibiotic to the gastric mucosal layer. The complex has a structure in which the non-absorbable antibiotic is inserted into clay mineral. The complex of the non-absorbable antibiotic and the clay mineral is orally administered to reach the stomach through the esophagus and then attached to the gastric mucosal layer to release the antibiotic (FIG. 2). That is, the eradication is not performed by a process in which the complex of the present disclosure is orally administered to reach the stomach through the esophagus and then the antibiotic is dissolved in the stomach, the dissolved antibiotic is moved to the intestine, absorbed in the intestine, and moved over the blood vessel, and released to the gastric mucosa to eradicate H. pylori.

The clay mineral of the present disclosure can be applied to the gastric mucosal layer (mucosa) due to the plate-like structure and enables the targeted treatment on the gastric mucosal layer due to an excellent drug impregnation capacity. In addition, a minimum inhibitory concentration (MIC) can be effectively maintained to an affected area, and the patient's burden on the antibiotics is reduced.

The complex of the non-absorbable antibiotic and the clay mineral as claimed may be prepared using general methods known in the art. The manufacturing method of the complex of binding the clay mineral and the drug has been disclosed until now through a plurality of thesis and the like, and those skilled in the art may manufacture the complex of the present disclosure by appropriately using the method. For example, the complex of the non-absorbable antibiotic and the clay mineral of the present disclosure may be manufactured by a method of Korean Patent Registration No. 10-1541876 in which the whole thereof is included as the reference of the present disclosure. In the case, the complex of the present disclosure may be manufactured by using the manufacturing method of the complex, including supplying a dispersion solution of clay mineral microparticles having an expandable lattice structure and an antibiotic solution to a first space and a second space separated from each other by an ion-exchange membrane, respectively, and maintaining the solutions for 1 hour; and removing the antibiotic solution from the second space and then supplying an antibiotic ion washing solution to the second space.

### β-lactam antibiotic

The complex of the non-absorbable antibiotic and the clay mineral of the present disclosure may be administered to a subject together with a β-lactam antibiotic and/or a gastric acid-suppressive agent. Therefore, the pharmaceutical composition and the kit of the present disclosure may further comprise a β-lactam antibiotic and a gastric acid-suppressive agent in addition to the complex of the non-absorbable antibiotic and the clay mineral.

The β-lactam antibiotic may be penicillin, methicillin, ampicillin, amoxicillin, cephalosporin, carbapenem, and the like, preferably, amoxicillin.

### Gastric acid-suppressive agent

The complex of the non-absorbable antibiotic and the clay mineral of the present disclosure may be administered to a subject together with a β-lactam antibiotic and/or a gastric acid-suppressive agent. The gastric acid-suppressive agent of the present disclosure is a proton pump inhibitor. The gastric acid-suppressive agent of the present disclosure uses general gastric acid-suppressive agents and is not particularly limited. For example, the gastric acid-suppressive agent of the present disclosure may be omeprazole, esomeprazole, rabeprazole, lansoprazole, pantoprazole, and the like. When the subject using the pharmaceutical composition or the kit of the present disclosure has a weak stomach or hyperacidity, the gastric acid-suppressive agent may be administered in combination with the complex of the non-absorbable antibiotic and the clay mineral.

### Kit

The kit of the present disclosure comprises a complex of a non-absorbable antibiotic and a clay mineral. Preferably, the kit of the present disclosure further comprises a β-lactam antibiotic and/or a gastric acid-suppressive agent. The kit of the present disclosure is an orally-administered kit for eradicating clarithromycin-resistant H. pylori.

### Pharmaceutical composition

The pharmaceutical composition of the present disclosure comprises a complex of a non-absorbable antibiotic and a clay mineral. The pharmaceutical composition of the present disclosure further comprises a β-lactam antibiotic and a gastric acid-suppressive agent.

In the pharmaceutical composition as claimed, the complex of the non-absorbable antibiotic and the clay mineral is orally administered to reach the stomach through the esophagus and then attached to the gastric mucosal layer to release an antibiotic. The pharmaceutical composition has the urease inhibitory activity and may be a pharmaceutical composition for preventing or treating gastrointestinal diseases caused by clarithromycin-resistant Helicobacter pylori.

The pharmaceutical composition of the present disclosure is a pharmaceutical composition for preventing, alleviating or treating gastrointestinal diseases caused by clarithromycin-resistant Helicobacter pylori. The disease caused by Helicobacter pylori may be gastrointestinal injury, gastritis, gastric ulcer, duodenal ulcer, gastritis, gastric cancer or MALT lymphoma.

The pharmaceutical composition of the present disclosure may comprise the complex of the non-absorbable antibiotic and the clay mineral, and the β-lactam antibiotic and the gastric acid-suppressive agent in 0.01 to 80 wt%, preferably 0.02 to 65 wt%. However, the amount may be increased or decreased according to the needs of a user, and may be appropriately increased or decreased depending on a situation, such as age, diet, nutrition condition, and disease progression. In the pharmaceutical composition of the present disclosure, a ratio, that is, a composition of the complex of the non-absorbable antibiotic and the clay mineral, and the β-lactam antibiotic and/or the gastric acid-suppressive agent may be appropriately determined by those skilled in the art.

The pharmaceutical composition of the present disclosure is orally administered and may be used in the form of general pharmaceutical preparations. Preferred pharmaceutical preparations include orally-administered preparations such as tablets, hard or soft capsules, liquids, suspensions, syrups, and chewing tablets, and these pharmaceutical preparations may be prepared using general pharmaceutically acceptable carriers, for example, in the case of orally-administered preparations, excipients, binders, disintegrants, lubricants, solubilizers, suspensions, preservatives, or extenders.

The dose of the pharmaceutical composition of the present disclosure may be determined by experts according to various factors such as the condition, age, sex, and complications of patients, but may be generally administered in a dose of 0.1 mg to 10 g, preferably 10 mg to 5 g per 1 kg of adult. Further, the pharmaceutical composition is contained in a daily dose or 1/2, 1/3, or 1/4 dose thereof, and may be administered 1 to 6 times a day. However, in the case of long-term ingestion for the purpose of health and hygiene or health regulation, the amount may be used below the range and an attending physician may appropriately control the amount.

### Diseases caused by Helicobacter pylori

The pharmaceutical composition and the kit of the present disclosure may be to prevent, alleviate, or treat diseases caused by clarithromycin-resistant Helicobacter pylori. The disease caused by Helicobacter pylori may be gastrointestinal injury, gastritis, gastric ulcer, duodenal ulcer, gastritis, gastric cancer or MALT lymphoma. The Helicobacter pylori may be general Helicobacter pylori and antibiotic-resistant Helicobacter pylori. At this time, the antibiotic-resistant Helicobacter pylori is clarithromycin-resistant Helicobacter pylori. The pharmaceutical composition and the kit of the present disclosure have the eradication activity on the clarithromycin-resistant Helicobacter pylori.

### Treating method or eradicating method

The present disclosure relates to a method for eradicating clarithromycin-resistant H. pylori comprising administering the pharmaceutical composition of the present disclosure to a subject. Further, the present disclosure relates to a method for eradicating antibiotic-resistant H. pylori comprising administering the pharmaceutical composition of the present disclosure to a subject. Further, the present disclosure relates to a method for preventing, alleviating or treating diseases caused by H. pylori comprising administering the pharmaceutical composition of the present disclosure to a subject. Further, the present disclosure relates to a method for preventing, alleviating or treating diseases caused by clarithromycin-resistant H. pylori comprising administering the pharmaceutical composition of the present disclosure to a subject.

The subject may be mammals diagnosed as infected with H. pylori, or having the risk of H. pylori infection. Further, the subject may be mammals diagnosed as infected with antibiotic-resistant H. pylori, or having the risk of antibiotic-resistant H. pylori infection. The mammals include humans. Therefore, the treating method of the present disclosure can treat subjects who have been infected with clarithromycin-resistant H. pylori or may be infected with clarithromycin-resistant H. pylori to have a larger width of subjects to be treated than conventional drugs for eradicating H. pylori. Further, the eradicating method of the present disclosure can also eradicate antibiotic-resistant H. pylori to have a larger width of subjects to be eradicated than conventional drugs for eradicating H. pylori.

### Antibiotic-resistant Helicobacter pylori

The complex of the non-absorbable antibiotic and the clay mineral or the pharmaceutical composition comprising the complex of the present disclosure has the eradication activity on clarithromycin-resistant H. pylori. The antibiotic-resistant Helicobacter pylori is preferably clarithromycin-resistant Helicobacter pylori. The antibiotic-resistant Helicobacter pylori may be Helicobacter pylori having monoresistance to clarithromycin. The antibiotic-resistant Helicobacter pylori may be Helicobacter pylori having simultaneous resistance to clarithromycin and metronidazole or having resistance to one or more antibiotics selected from metronidazole, tetracycline, levofloxacin, and the like in addition to clarithromycin, that is, multiple resistance to plurality of antibiotics. Amoxicilin resistance is less than 10%, while recently, the resistance to clarithromycin of Helicobacter pylori tends to be increased, and a major cause of the failed treatment for eradicating H. pylori is clarithromycin resistance. Therefore, the complex of the non-absorbable antibiotic and the clay mineral or the pharmaceutical composition comprising the complex of the present disclosure having the eradication activity on clarithromycin-resistant Helicobacter pylori is particularly useful for subjects failed to conventional eradication treatment.

Advantages and features of the present disclosure, and methods for accomplishing the same will be more clearly understood from exemplary embodiments described in detail below with reference to the accompanying drawings. However, the present disclosure is not limited to the exemplary embodiments set forth below, and will be embodied in various different forms. The exemplary embodiments are just for rendering the disclosure of the present disclosure complete and are set forth to provide a complete understanding of the scope of the invention to a person with ordinary skill in the art to which the present disclosure pertains, and the present disclosure will only be defined by the scope of the claims.

### <Materials and Methods>

### <Experimental Ethics>

Animal experiments were approved by the Incheon National Center of Efficacy Evaluation for the Development of Health Products Targeting Digestive Disorders 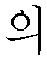 the Institutional Animal Care and Use Committee in Korea and conducted on rats. Mouse experiments were reviewed and approved by the Institutional Animal Care and Use Committee of Wongwang University School of Medicine. The mice were treated according to the guidelines and controls for animal use and management of the Wongwang University in Iksan, Korea and received any water and standard experimental diets.

### <Statistical analysis>

The data were represented by average ± SD, and experimental groups were compared using a nonparametric Mann-Whitney-U test. 95% CIs for the detection rate was obtained using a MINITAB statistical software program (Minitab Inc., PA, USA). If 95% CIs of the two values are not overlapped with each other, it was considered that the two values are considerably different. It was considered that P value < 0.05 is statistically important. The results were analyzed using Statistics Package for Social Science (SPSS 12.0 for Windows; SPSS Inc., Chicago, IL, USA).

### <Experimental Example 1> Eradication activity of antibiotic-clay mineral complex

### <1-1> Insertion of antibiotic

A gentamicin solution (2 mg/mL) was prepared using the United States Pharmacopeia (USP)-class gentamicin sulfate produced from Bio Basic Inc.

Ca-smectite was prepared by purifying bentonite in Gyeong-buk area in Korea. The gentamicin-smectite complex S-GEN (gentamicin-inserted smectite hybrid) was mixed with 250 ml of the gentamycin solution per gram of Ca-smectite and vigorously stirred for 24 hours to be prepared. Herein, the term of the gentamicin-smectite complex S-GEN (gentamicin-inserted smectite hybrid) is interchangeably used in combination with the term of S-GM. After mixing, the hybrid solution was dialyzed with 5 L of distilled water at 50°C for 8 hours, which was repeated 3 or 4 times until sulfate ions were not detected to PbCl₂. The hybrid powder was obtained by lyophilizing the dialyzed hybrid solution for 2 to 3 days. The amount of gentamicin released from the hybrid was determined by a batch-emission test, and at this time, 25 mL of a pH 1.2 solution was repeatedly added to the same 100 mg of the hybrid powder. The gentamicin concentration from a supernatant was measured using LC-MS. LC assays were performed using a Thermo Scientific ICS system. MS assay was performed using electronspray ionization and a Thermo Scientific MSQ Plus single-quadrupole mass spectrometer. The total amount of gentamicin released within 1 hour was confirmed as up to 5.0 mg per 100 mg of the hybrid.

Meanwhile, a netilmicin-smectite complex S-NET was prepared using netilmicin instead of gentamicin in the same manner.

### <1-2> Evaluation of eradication activity in vitro

Eradication activities of S-GEN and S-NET were evaluated. Helicobacter pylori was applied (red) in a medium and then a sample was made in a disk form and placed on the medium to measure the eradication effect. At this time, it was meant that if red disappeared around the disk, there was the eradication effect.

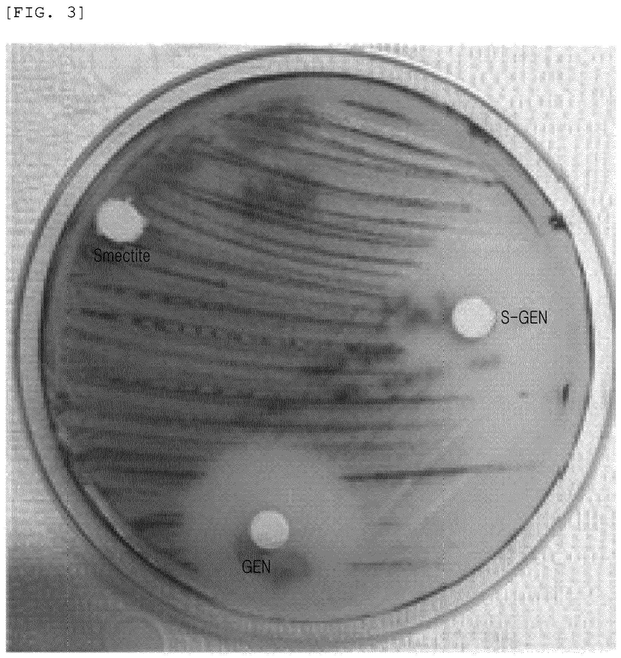

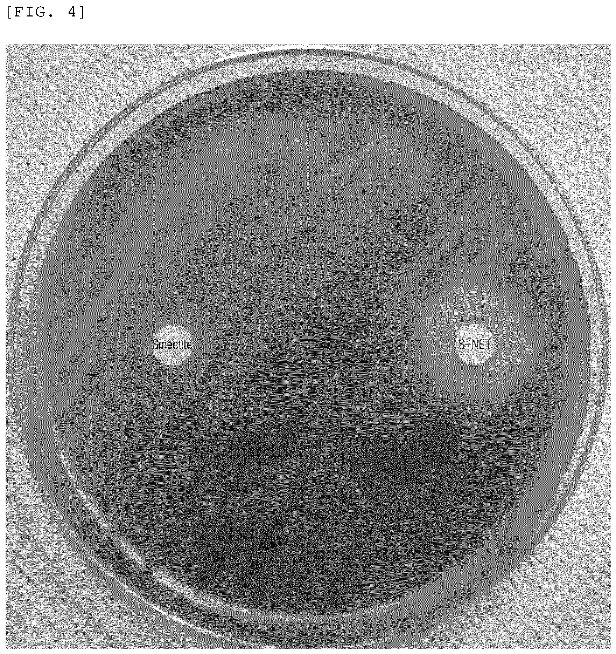

As a result, it was confirmed that the gentamicin-smectite composite (S-GEN) and the netilmicin-smectite complex (S-NET) had the eradication activity against Helicobacter pylori (FIGS. 3 and 4, upper left of FIG. 3: smectite, upper right of FIG. 3: S-GEN, lower of FIG. 3: gentamicin; left of FIG. 4: smectite, right of FIG. 4: S-NET) .

### <Experimental Example 2> Application on gastric mucosa in rats

7-week-old male Sprague-Dawley rats (weight 220 g ± 20%) were purchased from the Samtako Ltd. in Osan, Korea to test the application on the gastric mucosa. Rats were fasted for 24 hours before experiment. Two groups of 10 rats were taken with 10 mL/kg (150 mg/kg) of smectite or S-GEN and euthanized after 1 hour. For the analysis of the application efficiency of the gastric mucosa, the stomachs of the rats were resected and cut along a greater curvature and pinned. The stomach distribution ratio was calculated according to the following Equation 1. Stomach distribution ratio (%) = {Stomach distribution area (cm2)/Stomach entire area (cm2)} × 100

The ratio and the area were analyzed using Leica Application Suite V4 (Leica Microsystems Ltd., Korea). The stomach distribution ratio was represented by an average (± standard deviation).

### <Experimental Example 3> Anti-H. pylori effect in vivo

### <3-1> Inoculation of experimental animals

4-week-old male C57BL/6 mice were purchased from Japan SLC, Inc. in Shizuoka, Japan for evaluation of anti-H. pylori. The mice had five-week-old and a weight of 18 to 20 g at the time of the start of experiment. For inoculation, H. pylori SS1 was used. Bacteria were cultured in a Brucella blood agar (Merck, Germany) at 37°C for 72 hours under microaerophilic conditions (10% CO₂, 85% N₂ and 5% O₂). For anti-H. pylori evaluation in vivo, 80 mice were adapted for 1 week before the experiment.

After adapting, animals were fasted for 12 hours, 70 rats were infected with 0.5 mL of a 2.0 × 10⁹ cfu/mL H. pylori suspension and administered into the stomach through oral food intake every 48 hours, 3 times per week. The inoculation day was considered as 0 day and the next time was considered as 1 to 21 days. A non-infected group was used as a normal control group and received an equivalent volume of PBS and distilled water.

### <3-2> Treatment of experimental animals

The mice were divided into eight groups of 10 mice, and rested for one week after the last inoculation:
Group 1: Normal group of non-infected mice.
Group 2: Non-treated control group receiving distilled water.
Group 3: Treated with amoxicillin (14.25 mg/kg), clarithromycin (7.15 mg/kg), and gastric acid-suppressive agent (proton pump inhibitor) (PPI, omeprazole was used in all groups receiving 400 µmol/kg of PPI) and used as a positive control group.
Group 4: Treated with amoxicillin (14.25 mg/kg), gentamicin (4 mg/kg), and PPI (400 µmol/kg).
Group 5: Treated with amoxicillin (14.25 mg/kg), S-GEN (78 mg/kg), and PPI (400 µmol/kg).
Group 6: Treated with gentamicin (4 mg/kg) and PPI (400 µmol/kg).
Group 7: Treated with S-GEN (78 mg/kg) and PPI (400 µmol/kg).
Group 8: Treated with amoxicillin (14.25 mg/kg) and PPI (400 µmol/kg).

The treatments were conducted by oral administration once a day for 7 consecutive days. In order to confirm the serological position of H. pylori in infected mice, H. pylori immunoglobulin G (IgG) levels were confirmed with an ELISA kit (Cusabio Biotech Co., USA) before the treatment.

### <Experimental Example 4> Confirmation of bacteria

After 12 hours of last administration, the mice were euthanized and the stomach tissues were extracted. The gastric mucosa from the pylorus was bio-tested for a Campylobacter-like organism (CLO) test and PCR for H. pylori. In addition, 0.5 g of the stool per mouse was collected from the rectum and the colon, suspended with the same volume of distilled water, and filtered for H. pylori antigen (Ag) detection and H. pylori PCR in the stool.

### <4-1> CLO test

Gastric mucosa samples of the pylorus were analyzed with CLO kits (Asan Pharmaceutical Co., Seoul, Korea) and cultured at 37°C for 12 hours for testing the urease activity. The reaction (color change) was considered as negative in light yellow or positive in dark red. The reaction score was 0 to 3, wherein 0 represented no color change, 1 represented bright red, 2 represented bright purple, and 3 represented dark red.

### <4-2> H. pylori PCR in gastric mucosa

H. pylori DNAs were prepared using a beadbeater/phenol extraction method Kim B-J, Lee S-H, LyuM-A et al. Identification of mycobacterial species by comparative sequence analysis of the RNA polymerase gene (rpoB). J Clin Microbiol 1999; 37: 1714-20). A bacteria suspension was located in 2.0 mL screw-cap microcentrifugal tube filled with 200 µL of phenol, chloroform, and isoamyl alcohol (50:49:1) and 200 µL (paced volume) of glass beads (diameter, 1 mm; Biospec Products, Bartlesville, OK, USA). The tube was vibrated with a mini-beadbeater (Biospec Products) for 30 seconds and centrifuged for phase separation (12,000 g for 15 minutes). An aqueous phase was then moved to another clean tube, and added with 10 µL of 3 M sodium acetate and 250 µl of very cold absolute ethanol. In order to precipitate DNA, the mixture was maintained at - 20°C for 10 minutes. Harvested DNA pellets were dissolved in 60 µL of a Tris-EDTA buffer (pH 8.0) and were used as template DNA for PCR. The PCR was performed using AccuPower PCR Premix (Bioneer, Daejeon, Korea). After a first modification/activation step (95°C for 5 minutes), DNA (50 ng) was amplified using the following primers at a volume of 20 µL for 35 cycles of denaturation (94°C for 60 seconds), annealing (62°C for 60 seconds) and elongation (72°C for 90 seconds): H. pylori-specific ureA and ureC, sense 50-TGATGCTCCACTACGCTGGA-30 (SEQ ID NO: 1) and antisense 50-GGGTATGCACGGTTACGAGT-30 (SEQ ID NO: 2) (expected product 265 bp) (Kim YB, Kim ST, Lee SW et al. The influence of number of gastroscopic biopsy specimens on follow-up Campylobacter-like organism (CLO) test. Korean J Gastroenterol 2000; 35: 422-8), and GAPDH, sense 50-TGGGGTGATGCTGGTGCTG-AG-30 (SEQ ID NO: 3) and antisense 50-GGTTTCTC CAGGCGGCATGTC-30 (SEQ ID NO: 4) (expected product 497 bp) (Kundu P, Mukhopadhyay AK, Patra R et al. Cag pathogenicity is and independent up-regulation of matrix metalloproteinases-9 and -2 secretion and expression in mice by Helicobacter pylori infection. J Biol Chem2006; 281:34651-62). PCR products were analyzed by electrophoresis in a 1.5% agarose gel.

### <4-3> Detection of H. pylori antigen in mouse stool

A H. pylori antigen was evaluated using a commercially available SD Bioline H. pylori Ag kit (Standard Diagnostics, Inc.) according to the manufacturer's instructions. Samples (250 mg) were cultured with a dilution solution for 30 minutes at room temperature, and then 100 µL was located in a H. pylori antigen testing device. The results were checked after 15 minutes. A single red line represented a negative and a double red line represented a positive H. pylori result (Moon D-I, Shin E-H, Oh H-G et al. Usefulness of a Helicobacter pylori stool antigen test for diagnosing H, pylori infected C57BL/6 mice. Lab Anim Res 2013; 29: 27-32).

### <4-4> H. pylori PCR in mouse stool

Genomic DNA was extracted using a AccuPrep stool DNA extraction kit (Bioneer, Daejeon, Korea) according to the manufacturer's instructions (Lee J-U, Jung K, Kim O. Absence of vertical transmission of Helicobacter pylori in an experimental murine model. JVetSci 2006; 7: 225-8). A set of primers of SEQ ID NOs: 1 and 2 was used to amplify H. pylori-specific ureA and ureC (265 bp) (Kim YB, Kim ST, Lee SW et al. The influence of number of gastroscopic biopsy specimens on follow-up Campylobacter-like organism (CLO) test. Korean J Gastroenterol 2000; 35: 422-8). Template DNA (50 ng) and 20 pmol of each primer were added to a PCR mixture tube including 1 U of Taq DNA polymerase, 250 µM of each dioxynucleoside triophosphate, 50 mM Tris-HCl (pH 8.3), 40 mM KCl, 1.5 mM MgCl₂ and a gel loading dye. The volume was adjusted to 20 µL using distilled water. After initial denaturation at 95°C for 5 minutes, the reaction mixture was applied to 35 amplification cycles (at 94°C for 60 seconds, 62°C for 60 seconds, and 72°C for 90 seconds), and then elongated at 72°C for 10 minutes (GeneAmp 9700, Perkin Elmer, USA).

PCR products were electrophorous in a 1.5% agaros gel (Lee H-A, Park Y-S, KimO. Prevalence of Helicobacter species in feces of dogs using polymerase chain reaction analysis. Lab Anim Res 2007; 23:339-44).

### <Experimental Example 5> Quantification of inflammatory cytokines

The plasma was obtained for IL-8 and TNF-α analysis at 21 days by insertion of a heparinized microhaematocrit tube into the ophthalmic venous plexus of mice. Plasma IL-8 and TNF-α levels were measured using mouse ELISA kits (R&D Systems,Minneapolis,MN, USA).

### <Experimental Example 6> Anti-H. Pylori effect in vivo according to treatment condition of S-GEN

### <6-1> Inoculation of experimental animals

Male C57BL/6 mice were purchased from Japan SLC, Inc. in Shizuoka, Japan for evaluation of anti-H. pylori. 4-week-old mice were adapted and then infected with H. pylori SS1 3 times for 1 week, and maintained for 2 weeks. Thereafter, a test material was suspended according to a specified amount and administered orally at the same time every day by 10 ml per mouse kg. At this time, the test material was administered orally for 7 days, once a day.

### <6-2> Treatment of experimental animals

The mice were divided into five groups of 10 mice, and rested for one week after the last inoculation:
Normal control group: vehicle (no infection), PBS administration. received 10 mg/kg of distilled water.
Negative control group: vehicle (H. pylori infection), received 10 mg/kg of distilled water.
Test Example 1: (H. pylori infection) AMX (14.25 mg/kg/day), CLR (14.3 mg/kg/day), PPI (138 mg/kg/day)
Test Example 2: (H. pylori infection) AMX (14.25 mg/kg/day), S-GM (202 mg/kg/day), PPI (138 mg/kg/day) (at this time, Gentamicin 8 mg/kg)
Test Example 3: (H. pylori infection) AMX (14.25 mg/kg/day), S-GM (101 mg/kg/day)
AMX: amoxicillin
CLR: clarithromycin
S-GM: gentamicin-inserted smectite hybrid
PPI: gastric acid-suppressive agent

### <Experimental Example 7> Eradication effect of S-GM against antibiotic-resistant H. pylori

### <7-1> Evaluation of antibiotic resistant rate

Complexes of antibiotics (amoxicillin, clarithromycin, metronidazole, levofloxacin, and tetracyclin) and smectite which have been actually used for Helicobacter eradication treatment in current clinical trials were prepared and in vitro MIC for non-absorbable antibiotic candidate groups gentamicin, netilmicin, tobramycin, amikacin) to be used as a novel therapeutic agent was measured, and MIC50 and MIC90 were confirmed. To this end, 187 cultured positive clinical strains obtained from 1,265 patient samples were used.

### <7-2> Evaluation of eradication activity in vitro

With respect to a clarithromycin-resistant strain (MIC of CLR: 48 mg/L), the eradication activities of S-GM and clarithromycin were evaluated. Clarithromycin-resistant Helicobacter pylori was applied (red) in a medium and then a sample was made in a disk form and placed on the medium to measure the eradication effect. At this time, it was meant that if red disappeared around the disk, there was the eradication effect.

### <Results>

### Result of Experimental Example 2

### Application on gastric mucosal layer in rats

Experimental conditions of 10 mL/kg (150 mg/kg) were selected, and the experimental rats were euthanized after 1 hour of oral administration in consideration of a drug release time from the hybrid. S-GEN showed a 60.2% (± 14.3%) application rate.

### Result of Experimental Example 3

### Anti-H. Pylori effect in vivo

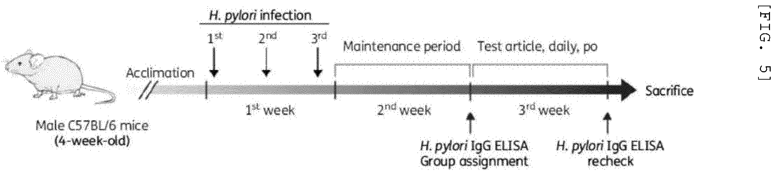

In order to evaluate an in-vivo effect of S-GEN against H. pylori, a H. pylori-infected model was made (FIG. 5). In addition, in order to confirm the H. pylori infection, H. pylori IgG levels were confirmed before and after treatment (Table 1: plasma concentration of H. pylori IgG in each group).

**[Table 1]**

| Group | Inoculation | | N | H. pylori IgG concentration | |
|---|---|---|---|---|---|
| | H. pylori infection | Treatment | | Before treatment | after treatment |
| Group1 | No | Distilled water | 10 | 0.26±0.01 | 0.25±0.00** |
| Group2 | Yes | Distilled water | 10 | 0.58±0.05a* | 0.74±0.01 |
| Group3 | Yes | AMX+CLR+PPI | 10 | | 0.33±0.01** |
| Group4 | Yes | AMX+GEN+PPI | 10 | | 0.35±0.01** |
| Group5 | Yes | AMX+S-GEN+PPI | 10 | | 0.32±0.01** |
| Group6 | Yes | GEN+PPI | 10 | | 0.40±0.01** |
| Group7 | Yes | S-GEN+PPI | 10 | | 0.34±0.01** |
| Group8 | Yes | AMX+PPI | 10 | | 0.35±0.01* |

| | | | | | |
|---|---|---|---|---|---|
| AMX: amoxicillin CLR: clarithromycin GEN: gentamicin | | | | | |

Data A represented by average ± standard deviation of 70 infected mice (Groups 2 to 8).
* Significant difference from Group 1 (P < 0.01)
** Significant difference from Group 2 (P < 0.01)

### Result of Experimental Example 4

### H. pylori PCR and CLO test of gastric mucosa

In mice, repeated intragastric inoculation of H. pylori made a positive response (red) in a CLO test of the gastric mucosa (Table 2: CLO test result as gastric mucosa after treatment).

**[Table 2]**

| Group^{a} | Percentage of negative animal by CLO test(95% CI^{b}) | CLO value |
|---|---|---|
| Group1 | 100 (72.2-100.0) | 0.0±0.0 |
| Group2 | 0 (0.0-27.6) | 3.0±0.0 |
| Group3 | 70 (39.7-89.2) * | 0.9±1.5* |
| Group4 | 60 (31.2-83.1) * | 1.2±1.6* |
| Group5 | 80 (49.0-94.3) * | 0.6±1.3* |
| Group6 | 50 (23.7-76.3) | 1.5±1.6 |
| Group7 | 60 (31.2-83.1) * | 1.2±1.6* |
| Group8 | 60 (31.2-83.1) * | 1.2±1.6* |

| | | |
|---|---|---|
| a Each group consists of 10 mice. b Incidence percentage (95% CI) was calculated by a MiniTab statistical program. * Significant difference from Group 2 (P < 0.05) | | |

The cure rate (100-positive responses) of the gastric mucosa were 70%, 60%, 80%, 50%, 60%, and 60% in Group 3 to 8, respectively. The CLO score of Group 5 was the lowest in H. pylori infected groups and was significantly lower than that of Group 2.

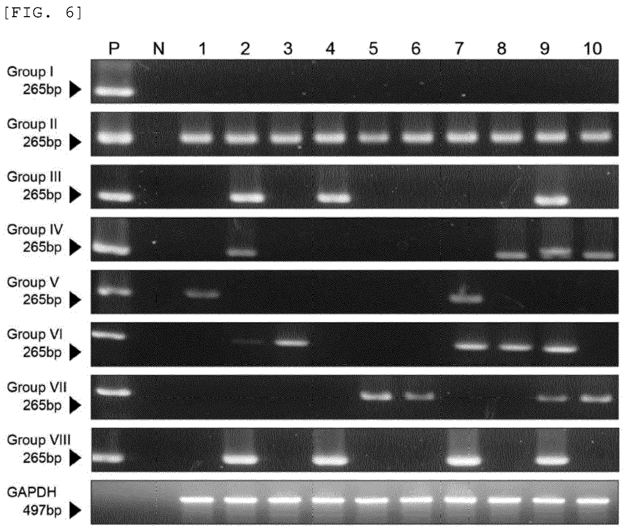

The PCR products of H. pylori-specific ureA and ureC (265 bp) were electrophored in a 1.5% agarose gel and visualized (FIG. 6).

The cure rate was the same as those confirmed by the CLO test.

### H. pylori antigen and PCR in stool of mouse

A stool antigen kit was used to detect H. pylori from the stool. Positive results in Group 2 and negative results in other groups were continuously observed.

H. pylori PCR was performed to evaluate therapeutic effects in H. pylori-infected mice (Table 3: PCR analysis of H. pylori in the stool after treatment). The cure rates were 90% and 100% in Group 3 (standard therapy) and Group 5 (treatment with amoxicillin/S-GEN/PPI), respectively.

**[Table 3]**

| Group^{a} | Percentage of negative animals by stool PCR(95% CI^{b}) |
|---|---|
| Group 1 | 100 (72.2-100.0) |
| Group2 | 0 (0-27.6) |
| Group3 | 90 (60.0-98.2) * |
| Group4 | 80 (49.0-94.3) * |
| Group5 | 100 (72.2-100.0) * |
| Group6 | 70 (39.7-89.2) * |
| Group7 | 80 (49.0-94.3) * |
| Group8 | 70 (39.7-89.2) * |

| | |
|---|---|
| a Each group consists of 10 mice. b Incidence percentage (95% CI) was calculated by a MiniTab statistical program. * Significant difference from Group 2 (P < 0.05) | |

### Result of Experimental Example 5

### Quantification of inflammatory cytokines

In order to confirm the effect of anti-H. pylori containing S-GEN on the production of cytokines, Plasma concentrations of inflammatory cytokines were measured in mice (Table 4). The levels of IL-8 and TNF-α in therapeutic groups were significantly lower than those in Group 2 (Table 4: Plasma concentration of IL-8 and TNF-α). The plasma levels of IL-8 and TNF-α in Group 5 were the lowest among treatment groups.

**[Table 4]**

| Group^{a} | IL-8 concentration(µg/ml) | TNF-α concentration(µg/ml) |
|---|---|---|
| Group1 | 3.73±0.82* | 16.14±4.99* |
| Group2 | 7.71±0.66 | 44.43±6.23 |
| Group3 | 4.12±0.45 * | 23.59±0.48** |
| Group4 | 3.98±0.21* | 23.20±2.52** |
| Group5 | 3.57±0.38* | 17.65±3.21** |
| Group6 | 4.24±0.42* | 24.30±1.84** |
| Group7 | 4.08±0.25* | 18.76±1.33** |
| Group5 | 4.52±0.36* | 22.13±3.59** |

The data was expressed as average ± standard deviation for 10 mice per group (µg/ml)
a Each group consists of 10 mice.
* Significant difference from Group 2 (P < 0.05)
** Significant difference from Group 2 (P < 0.01)

### Result of Experimental Example 6

Experimental animals treated in Experimental Example 6 were shown in Table 5 below.

**[Table 5]**

| | Cause of disease | Test substance | Dose(mg/kg) | Number of animals |
|---|---|---|---|---|
| Normal control group | PBS | Distilled water | 10 | 10 |
| Negative control group | *H.pylori* | Distilled water | 10 | 10 |
| Test group 1 | | AMX+CLR+PPI | 10 | 10 |
| Test group 2 | | AMX+S-GM+PPI | 10 | 10 |
| Test group 3 | | AMX+S-GM | 10 | 10 |

| | | | | |
|---|---|---|---|---|
| [1 time dose] (** dose once a day) AMX: amoxicillin CLR: clarithromycin S-GM: gentamicin)-inserted smectite hybrid PPI: gastric acid-suppressive agent Blood H. pylori IgG antibody titer | | | | |

### 1) Confirmation of H. pylori infection and measurement results for configuring groups

As a result of measuring H. pylori antibodies by an ELISA method to calculate an average and a standard deviation, the average value of a non-infection groups was measured as 0.04 ± 0.01 and the average value of the infectious groups was measured as 0.10 ± 0.10. H. pylori antibodies were statistically significantly increased to 150.0% in the infection groups as compared with the non-infection groups (P < 0.05) (Table 6).

**[Table 6]**

| | H. pylori IgG test |
|---|---|
| Non-infected group | 0.04±0.01 |
| infected group | 0.10±0.10* |

H. pylori antibody IgG test values after 1 week of infection.

The data were represented by average ± standard deviation. Statistical analysis was performed by Sigma plot statistic. * Comparison with non-infection group. *p < 0.05

### 2) Final H. pylori antibody IgG measurement results

As result of measuring H. pylori antibodies in the blood during autopsy to calculate the average and the standard deviation, it was measured in non-infected group (normal control group): 0.33 ± 0.13, infection control group, negative control group: 0.63 ± 0.29, Test group 1 (AMX + CLR + PPI administered group): 0.27 ± 0.07, Test group 2 (AMX + S-GM + PPI administered group): 0.19 ± 0.04, Test group 3 (AMX + S-GM administered group): 0.22 ± 0.06.

It was confirmed, in the negative control group infected with H. pylori, a 90.9% or more increase of H. pylori antibodies was measured compared to the normal control group which was the non-infected group non-infected with H. pylori, and H. pylori infection was statistically significantly maintained (p < 0.01). In Test group 1 (AMX + CLR + PPI administered group), a positive control substance, H. pylori antibodies were statistically significantly decreased to 57.1% as compared with the negative control group (p < 0.01). In Test group 2 (AMX + S-GM + PPI administered group) and Test group 3 (AMX + S-GM administered group) as test substance groups, H. pylori antibodies were statistically significantly decreased to 69.8% and 65.1% as compared with the negative control group, respectively (P < 0.01). In Test group 2 and Test group 3 as test substance groups, H. pylori antibodies were statistically significantly decreased to 29.6% and 18.5% as compared to Test group 1, which was a positive control group, and particularly, the reducing effect of Test group 2 was excellent (p < 0.01, p < 0.05).

### Stomach histopathologic analysis result

As a result of visually observing the stomach tissue extracted after autopsy, in a non-infected group (normal control group) and infected groups, a negative control group and Test groups 1 to 3, special lesions were not observed. As the histopathologic result, the findings observed in the stomach tissue were scored for the damage of the surface epithelium, inflammatory cell infiltration and submucosal edema. For each item, it was classified into 0 point if not observed, Mild 0.5 point, and Moderate 1 point.

As a result, in the normal control group, 0.10 ± 0.21 point in the damage of the surface epithelium, 0.15 ± 0.24 point in the inflammatory cell infiltration, and 0.10 ± 0.32 point in the submucosal edema were observed, and the total score as 0.35 ± 0.41 point. In the negative control group, 0.65 ± 0.34 point in the damage of the surface epithelium, 0.40 ± 0.32 point in the inflammatory cell infiltration, and 0.55 ± 0.37 point in the submucosal edema were observed, and the total score as 1.60 ± 0.81 point. In Test group 1 (AMX + CLR + PPI administered group), 0.30 ± 0.42 point in the damage of the surface epithelium, 0.40 ± 0.46 point in the inflammatory cell infiltration, and 0.60 ± 0.46 point in the submucosal edema were observed, and the total score as 1.30 ± 1.14 point. In Test group 2 (AMX + S-GM + PPI administered group), 0.30 ± 0.35 point in the damage of the surface epithelium, 0.15 ± 0.24 point in the inflammatory cell infiltration, and 0.35 ± 0.34 point in the submucosal edema were observed, and the total score as 0.80 ± 0.79 point. In Test group 3 (AMX + S-GM + PPI administered group), 0.60 ± 0.39 point in the damage of the surface epithelium, 0.40 ± 0.46 point in the inflammatory cell infiltration, and 0.65 ± 0.41 point in the submucosal edema were observed, and the total score as 1.65 ± 1.11 point (Table 7).

**[Table 7]**

| | | | Histopathologic score | | | |
|---|---|---|---|---|---|---|
| | | | Damage of the surface epithelium | Inflammatory cell infiltration | Submucosal edema | Total score |
| Normal control group | PBS | Distilled water | 0.10±0.21 | 0.15±0.24 | 0.10±0.32 | 0.35±0.41 |
| Negative control group | *H.pylori* | Distilled water | 0.65±0.34* * | 0.40±0.32* * | 0.55±0.37* * | 1.60±0.81* * |
| Test group1 | | AMX+CL R+PPI | 0.30±0.42# # | 0.40±0.46 | 0.60±0.46 | 1.30±1.14 |
| Test group2 | | AMX+S-G M+PPI | 0.30±0.35# # | 0.15±0.24# # | 0.35±0.34 | 0.80±.079# # |
| Test group3 | | AMX+S-G M | 0.60±0.39 | 0.40±0.46 | 0.65±0.41 | 1.65±1.11 |

The data were represented by average ± standard deviation. Statistical analysis was performed by Sigma plot statistic. * Comparison with non-infection group. ** p < 0.01, # comparison with negative control group, ##p < 0.01

As the result of comparing the scores for item "damage of the surface epithelium" for each group, it was observed that the negative control group was statistically significantly increased as compared with the normal control group (p < 0.01), and only Test group 1 (AMX + CLR + PPI administered group) and Test group 3 (AMX + S-GM + PPI administered group) were statistically significantly decreased to 53.8% and 53.8% as compared with the negative control group, respectively (Table 7).

As the result of comparing the scores for item "inflammatory cell infiltration" for each group, it was observed that the negative control group was statistically significantly increased as compared with the normal control group (p < 0.01), and only Test group 2 (AMX + S-GM + PPI administered group) was statistically significantly decreased to 62.5% as compared with the negative control group (Table 7).

As the result of comparing the scores for item "submucosal edema" for each group, it was observed that the negative control group was statistically significantly increased as compared with the normal control group (p < 0.01), and there was no statistically significant difference between the remaining groups (Table 7).

As the result of comparing the Total score for summing the scores of the histopathologic findings (damage of the surface epithelium, inflammatory cell infiltration and submucosal edema), it was observed that the negative control group was statistically significantly increased as compared with the normal control group (p < 0.01), and only Test group 1 (AMX + S-GM + PPI administered group) was statistically significantly decreased to 50.0% as compared with the negative control group (Table 7).

### Result of CLO rapid urease test

A CLO rapid urease test was conducted in the stomach tissue, and graphed to calculate the percentage of the number of positive samples for the total number of samples.

As a result, when a partially positive representing a moderate color was also regarded as a subject that showed the reaction to the treatment, the cure rates were 50% in Test group 1 (AMX + CLR + PPI administered group) as a standard therapy group, 70% in Test group 2 (AMX + S-GM + PPI administered group), and 10% in Test group 3 (AMX + S-GM administered group), and as a result, Test group 2 showed the best cure rate (Table 8).

**[Table 8]**

| | Cause of disease | Test substance | Ratio | | |
|---|---|---|---|---|---|
| | | | positive | partially positive | negative |
| Normal control group | PBS | Distilled water | 0/10 | 0/10 | 10/10 |
| Negative control group | *H.pylori* | Distilled water | 10/10 | 0/10 | 0/10 |
| Test group1 | | AMX+CLR+PPI | 5/10 | 0/10 | 5/10 |
| Test group2 | | AMX+S-GM+PPI | 3/10 | 1/10 | 6/10 |
| Test group3 | | AMX+S-GM | 9/10 | 0/10 | 1/10 |

### Result of Experimental Example 7

### Antibiotic-specific resistant rate

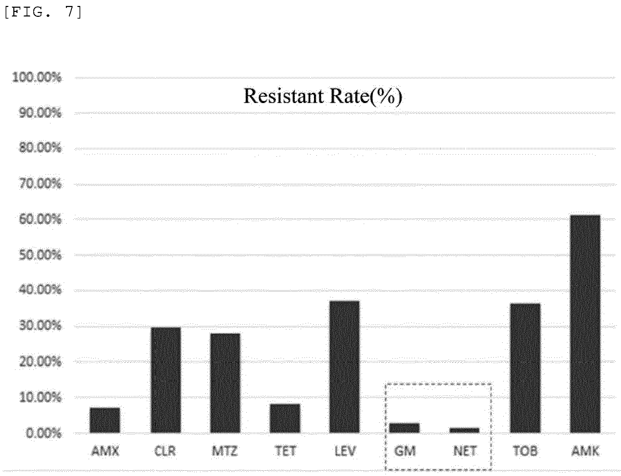

Clarithromycin had a very high resistant rate of 29.8%, and the resistant rate of levofloxacin, which was not frequently used for Helicobacter eradication in clinical trials, was the highest as 37.2%. Therefore, it was determined that clarithromycin was not suitable for use in manufacturing the clay mineral complex. In aminoglycoside-based antibiotics as candidate antibiotic groups to be used for the clay mineral complex, based on MIC 1 µg/ml, gentamicin and netilmicin showed relatively low MICs and resistant rates (2.78% and 1.43%), respectively, to be determined as an appropriate candidate antibiotic group. However, it was determined that tobramycin (TOB) had the resistant rate of 36.3% and amikacin (AMK) had the resistant rate of 61.3%, which were not suitable as a candidate antibiotic group (FIG. 7). AMX: amoxicillin, CLR: clarithromycin, MTZ: metronidazole, TET: tetracyclin, LEV: levofloxacin . GM: gentamicin, NET: netilmicin, TOB: tobramycin, AMK: amikacin.

### Eradication effect on clarithromycin-resistant strain

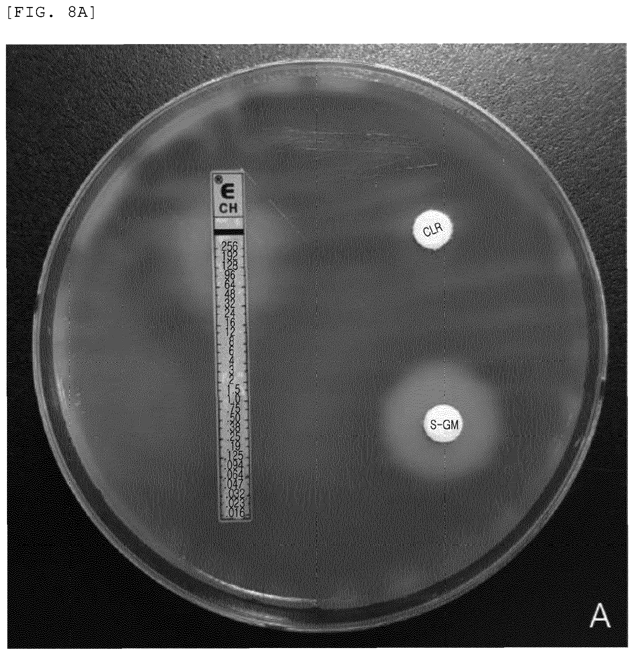

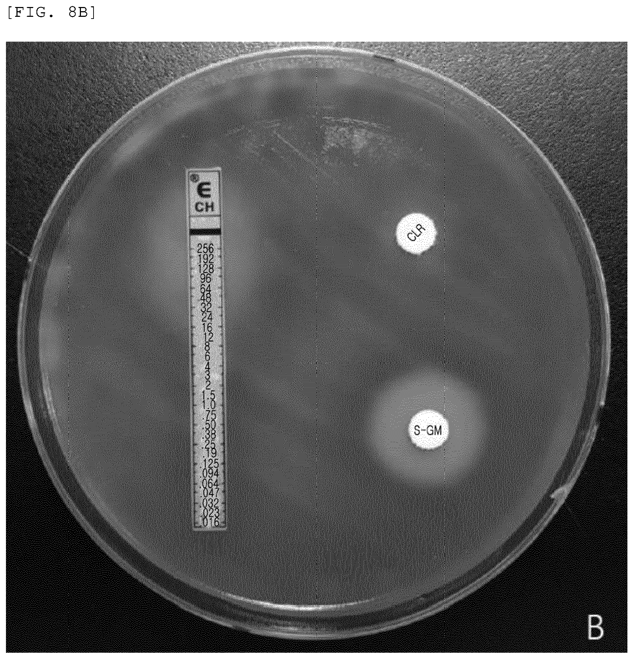

The eradication effect in vitro on clarithromycin-resistant H. pylori was evaluated. As the result of twice repeated tests, a gentamicin-smectite complex (S-GM) showed a clear zone around a disk. Therefore, it was confirmed that the gentamicin-smectite complex (S-GM) had the eradication activity against clarithromycin-resistant H. pylori. On the other hand, the clear zone was not shown around a clarithromycin (CLR) disk. The result of twice repeated tests was shown the same, and each result was shown in FIGS. 8A and 8B. (FIGS. 8A and 8B: Top: CLR: clarithromycin, Bottom: S-GEN: gentamicin-smectite complex).

### Review of experimental results

In the present disclosure, the effect of anti-H. pylori of S-GEN in a mouse model was evaluated. The experimental results of the present disclosure approved a significantly improved antimicrobial effect of S-GEN in reducing the H. pylori load in mouse's stomachs as compared with those of other therapies including a triple therapy as the current global standard in the H. pylori treatment.

The present inventors have developed S-GEN and evaluated whether S-GEN was effective in the treatment of H. pylori infection. H. pylori mainly lived within a mucosal layer attached to the gastric mucosa epithelial surface. Therefore, for effective treatment, S-GEN should be maintained on the stomach wall across the mucosal layer. In the present disclosure, S-GEN was well distributed on the stomach wall of the end, and 60.2% presence of S-GEN was observed, indicating that S-GEN maintenance was effectively made up to 1 hour. This result proposes that S-GEN may be used for direct eradication of H. pylori.

In the results of H. pylori PCR and CLO tests to the gastric mucosa after treatment, Group 5 showed the highest cure rate (80%) among the treated groups. In addition, the CLO value of Group 5 was the lowest among the treated groups (0.6 ± 1.3). An anti-H. pylori effect was clear in Group 5. In the case of a H. pylori PCR test on the stool after the treatment on the H. pylori infection, negative results were continuously observed only in Group 5. Therefore, the excellent anti-H. pylori activity applied by S-GEN triple treatment may be described as that a direct eradication effect thereof and the eradication effect which was applied for a longer time on the gastric mucosa as compared with other therapies of conventional antibiotics are further continued.

Interestingly, S-GEN-treated mice were H. pylori-infected, but had significantly reduced H. pylori-induced proinflammatory cytokines (IL-8 and TNF-α) as compared with non-treated mice. IL-8 pulls neutrophils to promote inflammation, and TNF-α induced the gastrin secretion together with IL-1β, which proposed the role of these cytokines in H. pylori-induced hypergastrinaemia and inflammatory responses. The immune response to H. pylori contributes to the onset of disease. Therefore, as observed herein, it is expected that a decrease in proinflammatory response reduces the inflammatory response responsible for permanizing the tissue damage. After S-GEN treatment, the initial removal of better anti-H. pylori effect and colonization than those accomplished by other therapies reduced the stomach inflammation.

In the present disclosure, treatment with S-GEN did not affect the mouse weight. The safety of smectite by oral administration was approved, and gentamicin was not absorbed through the gastric mucosa.

The present inventors have approved the strong antimicrobial activity of S-GEN against H. pylori and the long-term application effect of S-GEN in the gastric mucosal layer. The S-GEN treatment reduced the bacterial burden in vivo, compared to mice treated with a double or triple therapy including PPI or non-treated mice. In addition to a direct eradication effect, S-GEN has also helped to reduce inflammatory responses by inhibiting the production of proinflammatory cytokines.

In addition, it was confirmed that when administering S-GEN together with a β-lactam antibiotic and a gastric acid-suppressive agent, the eradication effect of clarithromycin-resistant Helicobacter pylori is significantly higher than the eradication effect when administering S-GEN together with only the β-lactam antibiotic.

In addition, it is determined that the resistant rate of H. pylori against clarithromycin, which is widely used in clinical trials, is high as 29.8%, the claimed formulation has the eradication effect even on clarithromycin-resistant H. pylori and S-GEN is useful even to patients who have no eradication effect on clarithromycin.

### <Explanation of sequence listing>

SEQ ID NO: 1 is a nucleotide sequence of a sense primer for amplification of H. pylori-specific ureA and ureC.

SEQ ID NO: 2 is a nucleotide sequence of an antisense primer for amplification of H. pylori-specific ureA and ureC.

SEQ ID NO: 3 is a nucleotide sequence of a sense primer for amplification of GAPDH.

SEQ ID NO: 4 is a nucleotide sequence of an antisense primer for amplification of GAPDH.

### [Industrial Availability]

According to the present disclosure, since the pharmaceutical composition and the kit include a platelike clay carrier, it is possible to be applied on the gastric mucosa and enable targeted therapy on the gastric mucosa. In addition, there are advantages of reducing the antibiotic destruction in an intra-gastric environment with low acidity and minimizing the impact on intestinal bacteria.

## Claims

1. An orally-administered pharmaceutical composition for eradicating Helicobacter pylori comprising a complex of a non-absorbable antibiotic and a clay mineral, a β-lactam antibiotic, and a gastric acid-suppressive agent,
wherein the non-absorbable antibiotic is gentamicin,
wherein the clay mineral is selected from a group consisting of montmorillonite, bentonite, beidellite, nontronite, saponite, and hectorite, and
wherein the pharmaceutical composition is for use in the eradication activity against clarithromycin-resistant Helicobacter pylori in a subject.

2. The pharmaceutical composition for use according to claim 1, wherein in the pharmaceutical composition, the complex of the non-absorbable antibiotic and the clay mineral is orally administered to reach the stomach through the esophagus and then attached to a gastric mucosal layer to release the antibiotic.

3. The pharmaceutical composition for use according to claim 1, wherein the pharmaceutical composition is a pharmaceutical composition for preventing or treating gastrointestinal diseases caused by Helicobacter pylori.

## Patentansprüche

1. Oral verabreichte pharmazeutische Zusammensetzung zur Ausrottung von Helicobacter pylori umfassend einen Komplex aus einem nicht resorbierbaren Antibiotikum und einem Tonmineral, ein ß-Lactam-Antibiotikum, und ein magensäurehemmendes Mittel,
wobei das nicht resorbierbare Antibiotikum Gentamicin ist,
wobei das Tonmineral aus einer Gruppe ausgewählt ist bestehend aus Montmorillonit, Bentonit, Beidellit, Nontronit, Saponit, und Hectorit, und
wobei die pharmazeutische Zusammensetzung zur Verwendung bei der Ausrottungsaktivität von Clarithromycin-resistentem Helicobacter pylori bei einem Subjekt ist.

2. Pharmazeutische Zusammensetzung zur Verwendung gemäß Anspruch 1, wobei in der pharmazeutischen Zusammensetzung, der Komplex aus dem nicht resorbierbaren Antibiotikum und dem Tonmineral oral verabreicht wird um durch die Speiseröhre den Magen zu erreichen und sich dann an einer Magenschleimhautschicht anzuheften um das Antibiotikum freizusetzen.

3. Pharmazeutische Zusammensetzung zur Verwendung gemäß Anspruch 1, wobei die pharmazeutische Zusammensetzung eine pharmazeutische Zusammensetzung zur Vorbeugung oder Behandlung von durch Helicobacter pylori verursachten gastrointestinalen Erkrankungen ist.

## Revendications

1. Composition pharmaceutique administrée par voie orale pour éradiquer Helicobacter pylori, comprenant un complexe d'un antibiotique non absorbable et d'un minéral argileux, d'un antibiotique β-lactame et d'un agent antisécrétoire gastrique,
dans laquelle l'antibiotique non absorbable est la gentamicine,
dans laquelle le minéral argileux est choisi dans un groupe consistant en la montmorillonite, la bentonite, la béidellite, la nontronite, la saponite et l'hectorite, et
dans laquelle la composition pharmaceutique est destinée à une utilisation dans l'activité d'éradication à l'encontre d'Helicobacter pylori résistant à la clarithromycine chez un patient.

2. Composition pharmaceutique pour une utilisation selon la revendication 1, dans laquelle, dans la composition pharmaceutique, le complexe de l'antibiotique non absorbable et du minéral argileux est administré par voie orale pour atteindre l'estomac en passant par l'œsophage, puis est fixé à la couche muqueuse gastrique pour libérer l'antibiotique.

3. Composition pharmaceutique pour une utilisation selon la revendication 1, dans laquelle la composition pharmaceutique est une composition pharmaceutique pour la prévention ou le traitement de maladies gastro-intestinales provoquées par Helicobacter pylori.
